# EUROPÄISCHE PATENTSCHRIFT

(11) **EP 1 208 239 B1**
(45) Veröffentlichungstag und Bekanntmachung des Hinweises auf die Patenterteilung: **29.03.2006**
(21) Anmeldenummer: 00962215.0
(22) Anmeldetag: 12.08.2000
(51) Int. Cl.: C12Q 1/68

(54) **VERFAHREN ZUM NACHWEIS UND ZUR QUANTIFIZIERUNG VON IN EINER FLÜSSIGKEIT BEFINDLICHEN ERSTEN BIOPOLYMEREN**
METHOD FOR DETECTING AND QUANTIFYING FIRST BIOPOLYMERS THAT ARE LOCATED IN A LIQUID
PROCEDE POUR DETECTER ET QUANTIFIER DES PREMIERS BIOPOLYMERES SE TROUVANT DANS UN LIQUIDE

(30) Priorität: 26.08.1999 DE 19940647
(43) Veröffentlichungstag der Anmeldung: 29.05.2002
(73) Patentinhaber: November Aktiengesellschaft Gesellschaft für Molekulare Medizin, 91056 Erlangen (DE)
(72) Erfinder: BERTLING, Wolf, D-91056 Erlangen (DE); SCHÜLEIN, Jürgen, D-91054 Erlangen (DE); HASSMANN, Jörg, D-91052 Erlangen (DE)
(74) Vertreter: Gassner, Wolfgang
(86) Internationale Anmeldenummer: PCT/DE2000/002757
(87) Internationale Veröffentlichungsnummer: WO 2001/016361

(56) Entgegenhaltungen:
- WO-A-93/20230
- WO-A-98/02399
- US-A- 5 312 527
- US-A- 5 567 301
- US-A- 5 871 918

### SEQUENZPROTOKOLL

<110> november Aktiengesellschaft
   Gesellschaft für Molekulare Medizin
<120> Verfahren zum Nachweis und zur Quantifizierung von in einer Flüssigkeit befindlichen Biopolymeren
<130> 401152GA
<140>
   <141>
<160> 2
<170> PatentIn Ver. 2.1
<210> 1
   <211> 21
   <212> DNA
   <213> Homo sapiens
<400> 1
   gccttcctaa ccattccctt a                                      21
<210> 2
   <211> 21
   <212> DNA
   <213> Homo sapiens
<400> 2
   taagggaatg gttaggaagg c                                      21

## Beschreibung

Die Erfindung betrifft ein Verfahren zum Nachweis und zur Quantifizierung von in einer Flüssigkeit befindlichen ersten Biopolymeren.

Nach dem Stand der Technik ist es bekannt, Polynukleotidsequenzen, wie DNA, durch voltammetrische Verfahren nachzuweisen. Dazu wird in der US 5,312,572 vorgeschlagen, redoxaktive Moleküle der Lösung zuzusetzen. Diese Moleküle binden im Falle der Hybridisierung an das aus den Polynukleotidsequenzen gebildete doppelsträngige Molekül. Das redoxaktive Molekül bewirkt ein meßbares Redoxsignal. Ein ähnliches Verfahren ist auch aus der US 5,871,918 bekannt.

Die WO 93/20230 beschreibt ein Verfahren zum Nachweis von in einer Flüssigkeit befindlichen ersten Biopolymeren, wobei zweite zu den nachzuweisenden ersten Biopolymeren eine spezifische Affinität besitzende Biopolymere an die Oberfläche einer ersten Elektrode gebunden sind und die erste Elektrode mit der Flüssigkeit in Kontakt ist. Beim bekannten Verfahren wird ein Spannung und/oder ein Strom über der ersten Elektrode angelegt und eine durch die Anlagerung der ersten Biopolymere an die zweiten Biopolymere bedingte unmittelbare Änderung der Spannung und/oder des Stroms gemessen.

Aus der WO 96/01836 ist ein sogenannter Chip zum Nachweis von Polynukleotidsequenzen bekannt. Auf dem Chip sind eine Vielzahl miniaturisierter Reaktionskavitäten vorgesehen. In jeder der Reaktionskavitäten ist eine besondere Polynukleotidsequenz gebunden. Beim Eintauchen des Chips in eine die nachzuweisende Polynukleotidsequenz enthaltende Lösung kommt es zur Hybridisierung mit einer der besonderen Polynukleotidsequen zen. Die Hybridisierung wird mittels Fluoreszenz nachgewiesen.

Auch die WO 95/12808 betrifft ein Nachweisverfahren mittels Chip. Dabei wird über den Chip nach Art einer Elektrode eine Spannung angelegt. Geladene in der Lösung befindliche Polynukleotidsequenzen können damit an der Oberfläche des Chips bzw. an den dort vorgesehenen miniaturisierten Reaktionsgefäßen angereichert werden.

Die Verfahren nach dem Stand der Technik sind aufwendig. Sie erfordern den Zusatz besonderer redoxaktiver Moleküle oder das Vorsehen aufwendig herzustellender Chips. Eine exakte Quantifizierung der nachzuweisenden Biopolymere ist mit den bekannten Verfahren nicht möglich. Es ist meist eine optische Detektion erforderlich. Das erhöht den apparativen Aufwand.

Aufgabe der Erfindung ist es, die Nachteile nach dem Stand der Technik zu beseitigen. Es soll insbesondere ein möglichst einfach und kostengünstig durchführbares Verfahren zum Nachweis und zur Quantifizierung von in einer Flüssigkeit befindlichen Biopolymeren angegeben werden.

Diese Aufgabe wird durch die Merkmale des Anspruchs 1 gelöst. Zweckmäßige Ausgestaltungen ergeben sich aus den Merkmalen der Ansprüche 2 - 14.

Das vorgeschlagene Verfahren ist einfach und schnell durchführbar. Die Änderung der Spannung und/oder des Stroms wird unmittelbar gemessen, d.h. das Vorsehen besonderer redoxaktiver Moleküle entfällt. Es ist insbesondere auch eine Quantifizierung der in der Flüssigkeit enthaltenden nachzuweisenden ersten Biopolymere auf einfache Weise möglich.

Die angelegte Spannung bzw. der Strom können veränderlich sein, z.B. kann es sich dabei um Wechselstrom handeln. Beim Schritt lit. b kann ein Gleichspannungssignal gemessen werden, wobei die Messung vorteilhafterweise als zyklovoltammetrische Messung geführt wird. In diesem Fall werden die Redoxprozesse der an den zweiten Biopolymeren angelagerten ersten Biopolymere und der Faradayische Elektronentransfer durch die Oberfläche der ersten Elektrode bei einer vorgegebenen Spannung oder in einem vorgegebenen Spannungsbereich genutzt.

Zum Nachweis und zur Quantifizierung des ersten Biopolymers wird vorteilhafterweise der gemessene Strom bzw. die gemessene Spannung über der Zeit aufgetragen und mindestens über einem Peak integriert. Die Integration erfolgt zweckmäßigerweise unter Abzug des Untergrunds. Aus dem sich bei der Integration ergebenden Wert kann die durch die Anlagerung der ersten Biopolymere übertragene Ladungsmenge ermittelt werden. Daraus läßt sich auf die Zahl der angelagerten ersten Biopolymere schließen. Es ist eine Eichung möglich. Die Konzentration der nachzuweisenden ersten Biopolymere in der Flüssigkeit kann bestimmt werden. Das vorgeschlagene Verfahren ist besonders einfach, weil hier - wie bei der herkömmlichen zyklischen Voltammetrie - nur zwischen der ersten Elektrode und einer Referenzelektrode zyklisch eine lineare Spannungsrampe durchfahren wird. Es ist nicht notwendig redoxaktive Moleküle zum Ladungstransport einzusetzen.

Der Stromfluß kann über eine dritte Elektrode bzw. eine Gegenelektrode gemessen werden. Beim Auftragen des gemessenen Stroms bzw. der gemessenen Spannung über der Zeit sind charakteristische Peaks beobachtbar, die der Anlagerung bzw. Adsorption oder einer Desorption der ersten Biopoymere zugeordnet werden können.

Eine weitere Ausgestaltung besteht darin, phasensensitiv ein Wechselstromsignal zu messen. Die Anlagerung der ersten Biopolymere an die an der ersten Elektrode endständig gebundenen zweiten Biopolymere verursacht eine Änderung der durch die Anlagerung und Abstoßung der geladenen Biopolymere bedingten Doppellagenkapazität. Die vorgeschlagene phasensensitive Messung ermöglicht die Ermittlung des kapazitiven Anteils des Wechselstromsignals. Die Kenntnis des kapazitiven Anteils läßt auf die Konzentration des nachzuweisenden ersten Biopolymers in der Flüssigkeit schließen.

Es ist in diesem Zusammenhang möglich, das Wechselstromsignal einem zyklischen Gleichstromsignal aufzuprägen. Nach einer weiteren Ausgestaltung des Verfahrens wird die Impedanz gemessen, indem die voltammetrischen Signale bei variierender Frequenz gemessen werden. Das ermöglicht eine unmittelbare Quantifizierung des Bedeckungsgrades mit nachzuweisenden ersten Biopolymeren auf der Oberfläche der ersten Elektrode.

Vorteilhaft ist es, vor dem Schritt lit. a die ersten Biopolymere durch Anlegen einer Spannung und/oder eines Stroms an der Oberfläche der ersten Elektrode anzureichern. Besonders wirkungsvoll ist die Anreicherung, wenn dabei zyklisch umgepolt wird. Dadurch werden nicht zu den zweiten Biopolymeren komplementäre Moleküle von der Oberfläche abgestoßen.

Nach einem weiterem Ausgestaltungsmerkmal ist vorgesehen, daß die erste Elektrode mit dem daran angereicherten ersten Biomolekülen aus der Flüssigkeit entfernt und, ggf. nach Ausführen eines Waschschritts, zur Messung mit einer definierten Meßlösung in Kontakt gebracht. Dadurch können störende Einflüsse durch in der Flüssigkeit eventuell befindliche weitere elektrochemisch wirksame Spezies weitgehend unterdrückt werden. Es findet gewissermaßen eine der Messung vorgeschaltete Aufreinigung statt.

Das zweite Biopolymer ist zweckmäßigerweise mit einem Ende kovalent oder über einen Linker an die Oberfläche der ersten Elektrode gebunden. Selbstverständlich können zur Bindung auch Spacermoleküle zwischengeschaltet sein. Die erste Elektrode ist zweckmäßigerweise aus einem der folgenden Materialien hergestellt: Kunststoff, Keramik, Glas oder Metall. Insbesondere Polycarbonate und Gold haben sich als Elektrodenmaterialien als besonders vorteilhaft erwiesen.

Unter den ersten und den zweiten Biopolymeren werden hier insbesondere Proteine, Peptide, DNA, RNA und dgl. verstanden. Das erste Biopolymer kann insbesondere eine zum zweiten Biopolymer komplementäre einzelstränige DNA oder RNA sein. Beim Schritt lit. b wird also vorzugsweise die durch die Hybridisierung der vorgenannten Biopolymere bedingte Änderung der Spannung und/oder des Stroms gemessen.

Das Verfahren wird anhand von Ausführungsbeispielen näher erläutert. Dazu zeigen die
- Fig. 1: ein zyklisches Voltammogramm und
- Fig. 2: die Änderung des Wechselstroms aufgetragen über der Frequenz und
- Fig. 3: eine schematische Ansicht eines Messaufbaus.

### Beispiel 1: Gleichspannung-Voltammetrische Messung

Ein Teil einer für das human growth hormone kodierenden DNA-Sequenz (HGH1) ist mit seinem 5'-Ende an eine aus Polycarbonat/Kohlefaser hergestellte erste Elektrode E1 bzw. Arbeitselektrode gebunden. Die erste Elektrode E1 taucht in eine Lösung ein, die 5fM/µl einer komplementären DNA-Sequenz (HGH1 comp.), 80mM TBE Puffer und 100mM NaCl Leitsalz enthält.

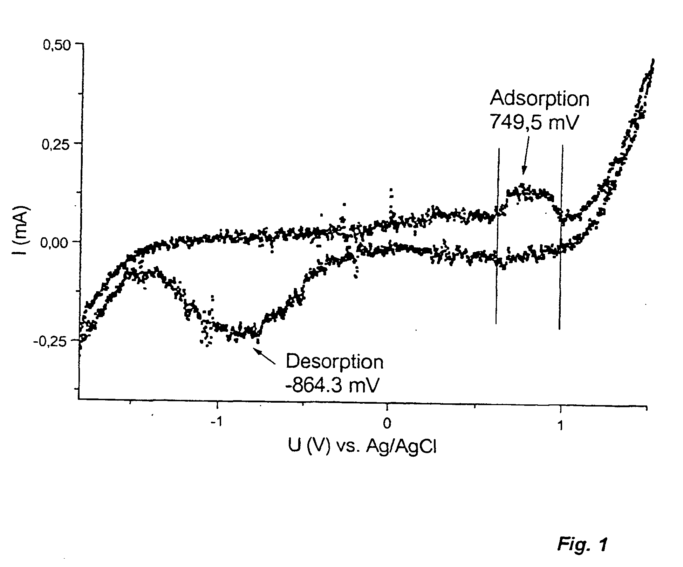

Eine Spannung wird linear mit einer Änderungsrate von 10mV/s von 0 auf 1.3V erhöht, dann zurück bis -1.3V und schließlich wieder bis auf 0V gefahren. Das dabei gemessene zyklische Voltammogramm ist in Fig. 1 gezeigt. Es sind zwei Peaks zu erkennen. Beim anodischen Potentialdurchlauf tritt ein erster Peak P1 bei U=749.5 mV gegen Ag/AgCl auf, beim kathodischen Potentialdurchlauf ergibt sich ein zweiter Peak P2 bei U= -864.3mV gegen Ag/AgCl. Diese Peaks treten nur auf, wenn sich zu den auf der Oberfläche der Elektrode fixierten ersten Biopolymeren komplementäre zweite Biopolymere in der Lösung befinden. Die Peaks P1, P2 sind ausschließlich auf Redoxprozesse zurückzuführen, die im vorliegenden Beispiel durch Hybridisation von HGH1 mit HGH1 comp. bedingt sind. Der im anodischen Bereich befindliche erste Peak P1 ist die Folge eines Redoxprozesses, der durch die Adsorption bzw. Anlagerung von HGH1 an HGH1 comp. bedingt ist. Der im kathodischen Bereich beobachtbare zweite Peak P2 kann mit der einer Desorption der hybridisierten Nukleinsäuren in Verbindung gebracht werden. Die zeitliche Integration des ersten Peaks P1 entspricht der durch die Elektrodenoberfläche transportierten Ladungsmenge. Daraus kann auf das Maß der Hybridisation auf der Oberfläche der ersten Elektrode geschlossen werden.

### Beispiel 2: Wechselstrom-Voltammetrische Messung

Es wird dieselbe Elektrode und Lösung wie im Beispiel 1 verwendet.

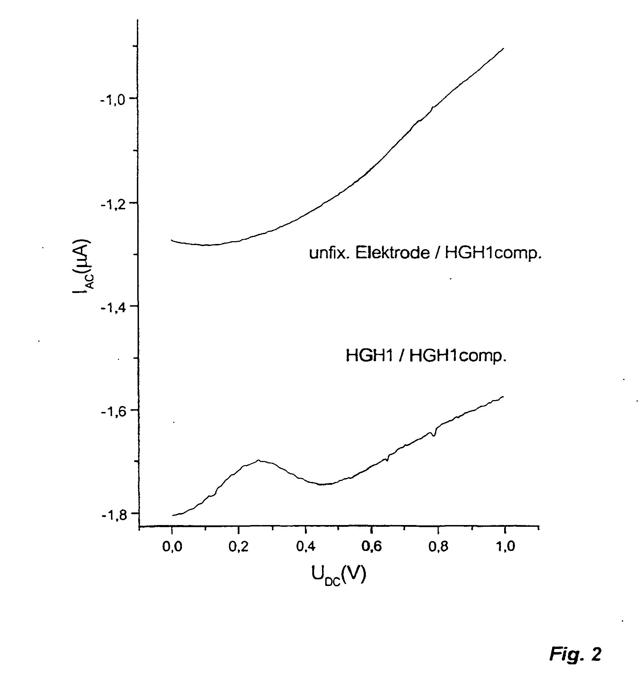

Ein Gleichspannungsscan von 0V bis 1V gegen Ag/AgCl wird angelegt und dieser Gleichspannung wird eine Wechselspannung von 250Hz überlagert. Der Wechselstrom wird in einer Phasenverschiebung von 90° zur Wechselspannung gemessen. Ist die erste Elektrode mit zu den in der Lösung befindlichen nachzuweisenden ersten Biopolymeren komplementären zweiten Biopolymeren belegt, so ergibt sich der in Fig. 2 gezeigte Peak bei 250mV. Dieser Peak ist durch eine Kapazitätsänderung bedingt, die durch den Transport negativer Ladungen an die Oberfläche der ersten Elektrode bei der Hybridisation hervorgerufen wird. Die Änderung in der Doppellagenkapazität resultiert in einem kapazitiven Stromfluß, der durch phasensensitive Messung des Wechselstromanteils detektiert werden kann. Die obere Kurve zeigt das Wechselstromsignal bei unbelegter erster Elektrode. Ein Peak ist hier nicht beobachtbar.

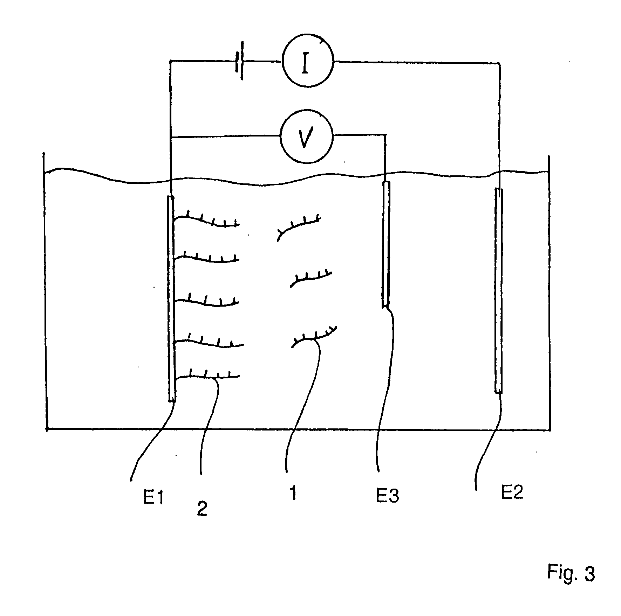

In Fig. 3 ist schematisch der Meßaufbau gezeigt. In eine Flüssigkeit tauchen eine erste Elektrode E1, eine zweite Elektrode E2 und eine dritte Elektrode E3 ein. Die erste Elektrode E1 ist die Arbeitselektrode. Daran sind zweite Biopolymere, z.B. DNA, mittels eines Linkers kovalent gebunden. In der Flüssigkeit befinden sich erste Biopolymere (1), die komplementär zu den zweiten Biopolymeren (2) sind. Die zweite Elektrode (E2) dient als Gegenelektrode, die dritte Elektrode (E3) als Referenzelektrode.

Das folgende Sequenzprotokoll gibt die Sequenzen von HGH1 und HGH1 comp. wieder.

## Patentansprüche

1. Verfahren zum Nachweis von in einer Flüssigkeit befindlichen ersten Biopolymeren (1), wobei zweite zu den nachzuweisenden ersten Biopolymeren (1) eine spezifische Affinität besitzende Biopolymere (2) an die Oberfläche einer ersten Elektrode (E1) gebunden sind und wobei die erste und mindestens eine zweite Elektrode (E2) mit der Flüssigkeit im Kontakt sind, mit folgenden Schritten:
a) Anlegen einer Spannung und/oder eines Stroms über der ersten (E1) und der zweiten Elektrode (E2),
b) Messung einer durch eine Adsorption oder Desorption der ersten Biopolymere (1) an die bzw. von den zweiten Biopolymere/n (2) bedingten unmittelbaren Änderung der Spannung und/oder des Stroms und
c) Nachweis durch Auftragen des gemessenen Stroms oder der gemessenen Spannung über der Zeit, wobei charakteristische Peaks beobachtbar sind, die der Adsorption oder Desorption der ersten Biopolymere zugeordnet werden können.

2. Verfahren nach Anspruch 1, wobei zur Quantifizierung der ersten Biopolymere (1) der gemessene Strom oder die gemessene Spannung über der Zeit aufgetragen und über mindestens einem Peak integriert wird.

3. Verfahren nach Anspruch 1 oder 2, wobei beim Schritt lit. b ein Gleichspannungssignal gemessen wird.

4. Verfahren nach Anspruch 3, wobei die Messung als zyklovoltammetrische Messung geführt wird.

5. Verfahren nach Anspruch 1 oder 2, wobei ein Wechselstromsignal phasensensitiv gemessen wird.

6. Verfahren nach Anspruch 5, wobei das Wechselstromsignal einem zyklischen Gleichstromsignal aufgeprägt ist.

7. Verfahren nach Anspruch 1 oder 2, wobei die Impedanz gemessen wird, indem die voltammetrischen Signale bei variierender Frequenz gemessen werden.

8. Verfahren nach einem der vorhergehenden Ansprüche, wobei vor dem Schritt lit. a die ersten Biopolymere (1) durch Anlegen einer Spannung und/oder eines Stroms an der Oberfläche der ersten Elektrode (E1) angereichert werden.

9. Verfahren nach Anspruch 8, wobei zyklisch umgepolt wird.

10. Verfahren nach Anspruch 8 oder 9, wobei die erste Elektrode (E1) mit den daran angereicherten ersten Biomolekülen (1) aus der Flüssigkeit entfernt und zur Messung mit einer definierten Meßlösung in Kontakt gebracht wird.

11. Verfahren nach einem der vorhergehenden Ansprüche, wobei das zweite Biopolymer (2) mit einem Ende kovalent oder über einen Linker an die Oberfläche der ersten Elektrode (E1) gebunden ist.

12. Verfahren nach Anspruch 11, wobei die erste Elektrode (E1) aus einem der folgenden Materialien hergestellt ist: Kunststoff, Keramik, Glas, Metall.

13. Verfahren nach einem der vorhergehenden Ansprüche, wobei das erste Biopolymer (1) eine zum zweiten Bioplymer (2) komplementäre einzelsträngige DNA oder RNA ist.

14. Verfahren nach Anspruch 13, wobei beim Schritt lit. b die durch die Hybridisierung der Biopolymere (1, 2) bedingte Änderung der Spannung und/oder des Stroms gemessen wird.

## Claims

1. A method for detecting first biopolymers (1) that are located in a liquid, where second biopolymers (2) which have a specific affinity to the first biopolymers (1) to be detected are bonded to the surface of a first electrode (E1), and where the first and at least one second electrode (E2) are in contact with the liquid, having the following steps:
a) application of a voltage and/or current across the first electrode (E1) and the second electrode (E2), and
b) measurement of a direct change in the voltage and/or current caused by an adsorption or a desorption of the first biopolymers (1) to or from the second biopolymer/s (2) and
c) detecting by plotting the measured current or the measured voltage against time, where characteristic peaks are observable which can be assigned to the adsorption or desorption of said first biopolymers.

2. A method as claimed in claim 1, where for the quantification of the first biopolymers (1), the measured current or the measured voltage ist plottet against time and integrated over at least one peak.

3. A method as claimed in claim 1 or 2, where in step b), a direct-voltage signal is measured.

4. A method as claimed in claim 3, where the measurement is carried out as a cyclovoltammetric measurement.

5. A method as claimed in claim 1 or 2, where an alternating-current signal is measured phase-sensitively.

6. A method as claimed in claim 5, where the alternating-current signal is superimposed on a cyclic direct-current signal.

7. A method as claimed in claim 1 or 2, where the impedance is measured by measuring the voltammetric signals at varying frequency.

8. A method as claimed in one of the preceding claims, where, before step a), the first biopolymers (1) are increased in concentration at the surface of the first electrode (E1) by application of a voltage and/or current.

9. A method as claimed in claim 8, where the polarity is reversed cyclically.

10. A method as claimed in claim 8 or 9, where the first electrode (E1) with the first biomolecules (1) increased in concentration at it is removed from the liquid and, for the measurement, brought into contact with a defined measurement solution.

11. A method as claimed in one of the preceding claims, where the second biopolymer (2) is bonded by means of one end to the surface of the first electrode (E1) via a covalent bond or via a linker.

12. A method as claimed in claim 11, where the first electrode (E1) is made of one of the following materials: plastic, ceramic, glass or metal.

13. A method as claimed in one of the preceding claims, where the first biopolymer (1) is a single-stranded DNA or RNA which is complementary to the second biopolymer (2).

14. A method as claimed in claim 13, where in step b), the change in voltage and/or current caused by hybridization of the biopolymers (1, 2) is measured.

## Revendications

1. Procédé pour la détection de premiers biopolymères (1) se trouvant dans un liquide, des deuxièmes biopolymères (2) ayant une affinité spécifique avec les premiers biopolymères (1) qui doivent être détectés, étant reliés à la surface d'une première électrode (E1), la première et au moins une deuxième électrode (E2) étant en contact avec le liquide, selon les opérations suivantes :
a) Application d'une tension et/ou d'un courant par l'intermédiaire de la première (E1) et de la deuxième électrode (E2),
b) Mesure par adsorption ou désorption des premiers biopolymères (1) aux ou des deuxièmes biopolymères (2) d'une modification immédiate conditionnelle de la tension et/ou du courant et
c) Détection par application du courant mesuré ou de la tension mesurée via le temps, des pics caractéristiques pouvant être observés, lesquels peuvent être attribués à l'adsorption ou à la désorption des premiers biopolymères.

2. Procédé selon la revendication 1, le courant mesuré ou la tension mesurée via le temps étant appliqué pour la quantification des premiers biopolymères (1) et intégré via au moins un pic.

3. Procédé selon la revendication 1 ou 2, un signal à tension continue étant mesuré à l'opération mentionnée au point b).

4. Procédé selon la revendication 3, la mesure étant effectuée comme mesure cyclovoltammétrique.

5. Procédé selon la revendication 1 ou 2, un signal à courant alternatif étant mesuré sensible aux phases.

6. Procédé selon la revendication 5, le signal à courant alternatif étant appliqué à un signal à courant continu cyclique.

7. Procédé selon la revendication 1 ou 2, l'impédance étant déterminée en mesurant les signaux voltammétriques à fréquence variée.

8. Procédé selon l'une des revendications précédentes, les premiers biopolymères (1) étant enrichis avant l'opération mentionnée au point a) par application d'une tension et/ou d'un courant à la surface de la première électrode (E1).

9. Procédé selon la revendication 8, une inversion de polarité cyclique étant effectuée.

10. Procédé selon la revendication 8 ou 9, la première électrode (E1) comprenant les premières biomolécules enrichies (1) étant retirée du liquide et mise en contact avec une solution de mesure définie pour la mesure.

11. Procédé selon l'une des revendications précédentes, le deuxième biopolymère (2) étant relié à une extrémité de manière covalente ou par l'intermédiaire d'un lieur à la surface de la première électrode (E1).

12. Procédé selon la revendication 11, la première électrode (E1) consistant en un des matériaux suivants: matière plastique, céramique, verre, métal.

13. Procédé selon l'une des revendications précédentes, le premier biopolymère (1) étant un ADN ou un ARN simple brin complémentaire au deuxième biapolymère (2).

14. Procédé selon la revendication 13, la modification de la tension et/ou du courant due à l'hybridation des biopolymères (1, 2) étant mesurée à l'opération mentionnée au point b).
